# EUROPEAN PATENT SPECIFICATION

(11) **EP 4 417 155 B1**
(45) Date of publication and mention of the grant of the patent: **23.09.2026**
(21) Application number: 24157796.4
(22) Date of filing: 15.02.2024
(51) Int. Cl.: A61B 18/20

(54) **LIGHT TREATMENT APPARATUS FOR TREATING VASCULAR LESIONS**
LICHTBEHANDLUNGSVORRICHTUNG ZUR BEHANDLUNG VON GEFÄSSLÄSIONEN
APPAREIL DE TRAITEMENT PAR LA LUMIÈRE POUR TRAITER DES LÉSIONS VASCULAIRES

(30) Priority: 15.02.2023 KR 20230020055
(43) Date of publication of application: 21.08.2024
(73) Proprietor: Lutronic Corporation, Goyang-si, Gyeonggi-do 10534 (KR)
(72) Inventor: KIM, Won Joong, 10533 Goyang-si, Gyeonggi-do (KR)
(74) Representative: Michalski Hüttermann & Partner mbB

(56) References cited:
- EP-A1- 0 363 221
- US-A1- 2009 093 799
- US-A1- 2011 196 355
- US-A1- 2015 305 811
- US-A1- 2019 083 810
- US-A1- 2020 251 873
- US-B1- 11 291 504
- US-B1- 8 202 268

## Description

### BACKGROUND OF THE INVENTION

### Field of the Invention

The disclosure relates to a light treatment apparatus for treating vascular lesions, and more particularly to a light treatment apparatus for treating vascular lesions, in which a pulsed laser is used to treat vascular lesions on skin.

### Description of the Related Art

Recently, there has been widely applied a technology of treating lesions by delivering energy to specific tissue in a human body to change the state of the tissue or remove the tissue. As the sources of such energy, lasers, flash lamps, radio frequency (RF) energy, microwaves, ultrasonic waves, etc. are employed, and various treatment apparatuses using these sources are being developed.

Among such treatment apparatuses, an apparatus using the laser or the like light as the energy source is widely used as a treatment apparatus for treating various lesions on skin, and such an apparatus has also been disclosed in Korean Patent No. 10-1269970. When skin tissue is irradiated with light, the light having specific wavelengths penetrates into the skin and is selectively absorbed in various tissues such as vascular, collagen and hair follicles located inside the skin according to the characteristics of the wavelengths. The absorbed light is converted into thermal energy inside those tissues, and thus the treatment is achieved in such a manner that the states of the tissues are changed by the thermal energy.

However, when vascular lesions on skin are treated by the light treatment apparatus, there is a limit because one apparatus is used in providing different parameters of treatment light even though the required parameters of the treatment light are varied depending on the size of a blood vessel, the location of a lesion, and the skin characteristics of a patient.

### Documents of Related Art

### (Patent Document)

Korean Patent No. 10-1269970 (May 24, 2012) and US 8202268 B1.

### SUMMARY OF THE INVENTION

An aspect of the disclosure is to provide a light treatment apparatus for treating vascular lesions, in which light is used to treat vascular lesions on skin, and the size of a blood vessel, the location of a lesion, the skin characteristics of a patient, etc. are taken into account to adjust the parameters of treatment light variously.

The invention is defined in the appended set of claims.

According to an embodiment of the disclosure, there is provided a light treatment apparatus for treating vascular lesions, including a light generating unit; and a setting unit configured to set a pulse pattern of treatment light generated in the light generating unit, wherein a single pulse mode for irradiating treatment light including a single pulse, and a sub-pulse mode for irradiating treatment light including a plurality of sub-pulses are set through the setting unit.

Here, the single pulse mode may be a treatment mode for treating relatively large blood vessels compared to the sub-pulse mode, and the sub-pulse mode may be a treatment mode for treating relatively small blood vessels.

The setting unit may be configured to adjust a pulse width of the treatment light, and an adjustable range for the pulse width of the treatment light in the single pulse mode may be configured to allow adjustment up to a relatively short pulse width compared to an adjustable range for the pulse width of the treatment light in the sub-pulse mode.

Specifically, the pulse width of the treatment light may be adjusted as a pulse width of 0.1 ms or more in the single pulse mode, and the pulse width of the treatment light may be adjusted as a pulse width of 5 ms or more in the sub-pulse mode.

In addition, the setting unit may be configured to adjust the pulse width of the treatment light, and a pulse width section selectable in the sub-pulse mode may include a first pulse width section where the pulse width is adjusted as sub-pulses that make up a treatment light pulse is changed in number, and a second pulse width section where the pulse width is adjusted as an off-time between the sub-pulses is changed while maintaining the number of sub-pulses that make up the treatment light pulse.

Alternatively, the setting unit may be configured to adjust the pulse width of the treatment light, and a pulse width section selectable in the sub-pulse mode may include a first pulse width section where the pulse width is adjusted as sub-pulses that make up a treatment light pulse is changed in number, and a third pulse width section where the pulse width is adjusted as an off-time between sub-pulse groups is changed while sub-pulses that make up the treatment light pulse are irradiated as a plurality of sub-pulse groups.

In addition, energy delivered to a treatment site per unit time may decrease as a pulse width increases in a pulse width having a predetermined value or more within a range of the pulse width adjustable in the sub-pulse mode.

Further, the sub-pulse mode may include a first sub-pulse mode with a plurality of sub-pulses having a pulse width within a range from 1 to 3 ms, and a second sub-pulse mode with a plurality of sub-pulses having a pulse width within a range from 100 to 700 µs.

A pulse width section selectable in the first sub-pulse mode may include a first pulse width section where the pulse width is adjusted as sub-pulses that make up a treatment light pulse is changed in number, and a second pulse width section where the pulse width is adjusted as an off-time between the sub-pulses is changed while maintaining the number of sub-pulses that make up the treatment light pulse.

Here, energy delivered to a treatment site per unit time in the first pulse width section may be maintained within a preset range, and energy delivered to a treatment site per unit time in the second pulse width section may decrease as the pulse width increases.

In addition, a pulse width section selectable in the second sub-pulse mode may include a first pulse width section where the pulse width is adjusted as sub-pulses that make up a treatment light pulse is changed in number, and a third pulse width section where the pulse width is adjusted as an off-time between sub-pulse groups is changed while sub-pulses that make up the treatment light pulse are irradiated as a plurality of sub-pulse groups.

In addition, energy delivered to a treatment site per unit time in the first pulse width section may be maintained within a preset range, and energy delivered to a treatment site per unit time in the third pulse width section may decrease as the pulse width increases.

### BRIEF DESCRIPTION OF THE DRAWINGS

FIG. 1 is a perspective view of a light treatment apparatus for treating vascular lesions according to an embodiment of the disclosure,
FIG. 2 is a block diagram schematically showing major components in FIG. 1,
FIG. 3 is a circuit diagram showing the configuration of a light generating unit in FIG. 2,
FIG. 4 is a graph showing the pulse structures of treatment light according to a plurality of treatment modes,
FIG. 5 is a graph showing the pulse structures of treatment light according to the pulse widths in a single pulse mode of FIG. 4,
FIG. 6 is a graph showing the pulse structures of treatment light according to the pulse widths in a first sub-pulse mode of FIG. 4, and
FIG. 7 is a graph showing the pulse structures of treatment light according to the pulse widths in a second sub-pulse mode of FIG. 4.

### DETAILED DESCRIPTION OF THE INVENTION

Hereinafter, a laser apparatus for vascular treatment according to embodiments of the disclosure will be described in detail with reference to the accompanying drawings. In the following description, a position relationship between components will be in principle based on the accompanying drawings. In addition, for convenience of description, the structure according to the disclosure may be simplified or exaggerated as necessary in the accompanying drawings. Therefore, the disclosure is not limited to the accompanying drawings, but may also be implemented by adding, changing, or omitting various devices.

Hereinafter, the term 'light treatment apparatus' refers to an apparatus used for light irradiation to improve the state of lesion or tissue, and may include all of apparatuses that treats mammals as well as humans.

Hereinafter, the term 'tissue' refers to a set of cells that make up various body organs of animals including humans, and may include various tissues that make up various internal organs of the body, such as skin tissue.

Hereinafter, the term 'light treatment apparatus for treating vascular lesions' includes a light treatment apparatus for regarding a blood vessel as a treatment area or treating vascular lesions. Here, the light treatment apparatus for treating vascular lesions may be configured to irradiate internal blood vessels with treatment light directly, and may also be configured to irradiate the internal blood vessels with the treatment light via surface tissue such as skin from the outside.

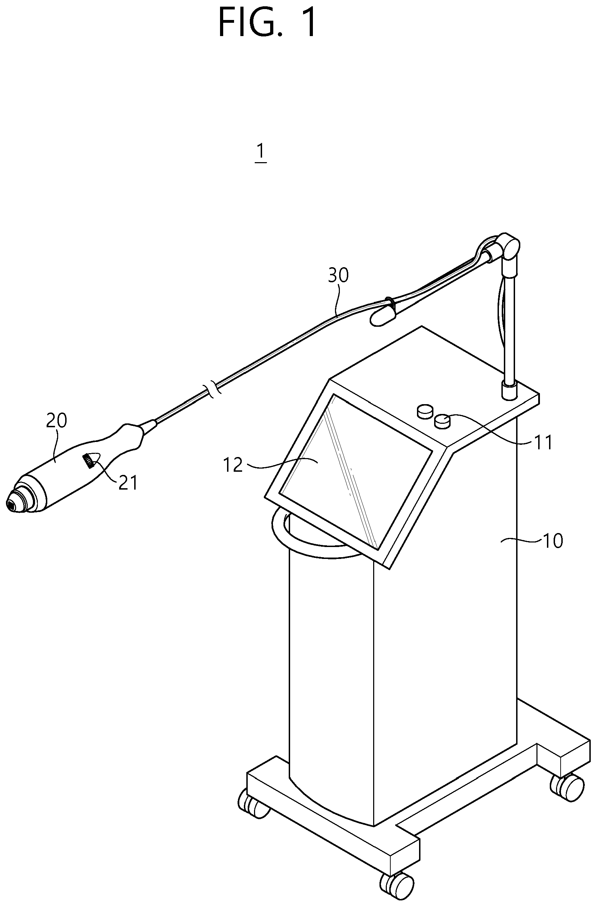

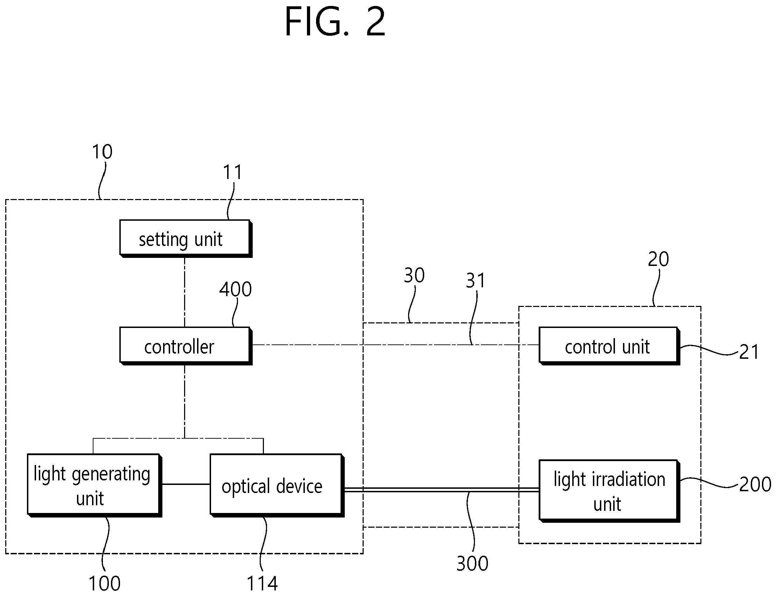

Below, the light treatment apparatus according to an embodiment of the disclosure will be described with reference to FIGS. 1 and 2. FIG. 1 illustrates the light treatment apparatus according to an embodiment of the disclosure. As shown in FIG. 1, a light treatment apparatus 1 according to an embodiment may include a main body 10, a handpiece 20, and a connector 30 for connecting the main body 10 and the handpiece 20.

The main body 10 forms a main framework of the light treatment apparatus, and is internally provided with various components. For example, the main body 10 is internally provided with a light generating unit 100 that generates the treatment light, and a plurality of optical devices disposed to form a path where the treatment light generated in the light generating unit is transmitted to a handpiece 20. On the outer surface of the main body 10, a setting unit 11 for setting a treatment mode, and a display 12 for showing various pieces of information to a user may be provided.

The handpiece refers to a component that receives the treatment light generated in the light generating unit and irradiate a treatment site with light. The handpiece is shaped to be held in a user's hand. A user may change the treatment site to perform treatment while holding the handpiece in his/her hand. At the end of the handpiece, a light irradiation unit may be provided to transmit the treatment light to the treatment site. On the outer surface of the handpiece, a control unit may be provided to control the operations of the handpiece or treatment operations. Further, the handpiece may additionally include a cooler (not shown) to protect a patient's skin from thermal damage during the treatment. In addition, the handpiece may additionally include a displacement sensor (not shown) for detecting the movement displacement of the handpiece during the treatment, or a temperature sensor (not shown) for detecting the temperature of a skin surface during the treatment.

The connector 30 refers to a component that connects the main body and the handpiece. The connector 30 may be internally provided with a light transmission portion 300 such as an optical fiber that forms an optical path from the light generating unit 100 to a light irradiation unit 200, and a signal line 31 that transmits various control signals from a controller 400 of the main body, a control unit 21 of the handpiece, etc.

FIG. 2 is a block diagram schematically showing the major components in FIG. 1. Below, the components of FIG. 1 will be described in more detail with reference to FIG. 2.

As shown in FIG. 2, the main body 10 is provided with the light generating unit 100. The light generating unit 100 includes a light source for generating the treatment light. As the light source, there may be used various known light sources such as a laser resonator, a laser diode, a dye laser, and a light emitting diode (LED). For example, the light generating unit 100 according to an embodiment includes a resonator 110 capable of oscillating laser.

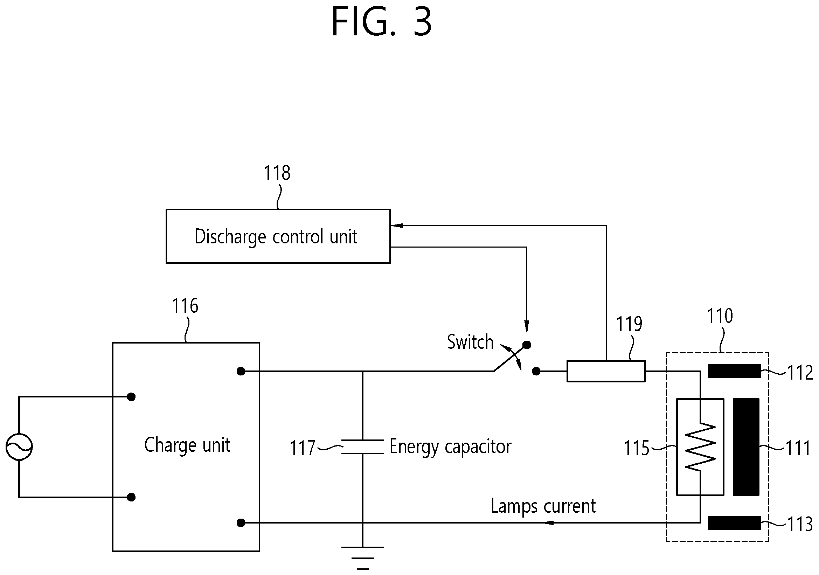

FIG. 3 is a circuit diagram showing the configuration of the light generating unit in FIG. 2. As shown in FIG. 3, the resonator 110 includes a laser medium 111, and reflection members 112 and 113 provided at both ends of the laser medium 111. In addition, there may be various optical devices such as a shutter, and a lens. The laser medium 111 is excited by an adjacent flash lamp 115, and the excited light is amplified while reciprocating inside the resonator, thereby generating laser light. An electric circuit connected to the flash lamp 115 includes a charge unit 116 for charging a capacitor 117 with electric energy, and a discharging control unit 118 for selectively opening and closing the path where current flows from the capacitor 117 to the flash lamp 115. Further, a current sensor 119 is provided on the circuit in which current flows, and the circuit is controlled in consideration of this. Through this circuit, the current is transmitted to the flash lamp 115, and the flash lamp selectively blinks to oscillate the laser medium 111. Therefore, the waveform of the current transmitted to the flash lamp is controlled by electric circuit control, thereby adjusting the pulse pattern of light generated in the light generating unit 100.

In FIG. 3, the structure of the light generating unit using the flash lamp and the resonator is described by way of example, but the disclosure is not limited thereto. Besides, various light sources capable of generating the treatment light in the form of pulses may be used.

The light generating unit 100 generates light varied in wavelength depending on the types of the laser medium, and thus the laser medium may be selected in consideration of the treatment sites and the treatment purposes. For example, the light generating unit according to an embodiment may employ an neodymium-doped yttrium aluminum garnet (Nd:YAG) laser medium.

In this case, the light generating unit 100 may generate the treatment light having a wavelength within a range from 1060 nm to 1070 nm. The treatment light generated by a long pulsed Nd:YAG laser and having a wavelength of 1064 nm is less absorbed by melanin and penetrates deeply into skin. Therefore, the treatment light of the above-mentioned wavelength range may be used in treating vascular lesions having a diameter of 4 mm or below, such as telangiectasia caused by the dilatation of blood vessels, and leg veins. Further, the treatment light of the above-mentioned wavelength range is well absorbed by methemoglobin and deoxyhemoglobin, i.e., main chromophores of blue vascular lesions, and thus useful to treat the blue vascular lesions. Further, when the pulse width is lengthened to apply low energy slowly during the treatment, it is possible to make the blood vessels coagulate while avoiding the rupture of the blood vessels, and purpura and pigmentation that appear after laser treatment.

Alternatively, the light generating unit may use a KTiOPO₄ (KTP) nonlinear crystal to convert the wavelength of the light output from the Nd:YAG laser, thereby generating the treatment light having a wavelength within a range from 530 nm to 535 nm. In particular, the treatment light having a wavelength of 532 nm is excellent in being absorbed by oxyhemoglobin, and thus effective in treating vascular lesions.

Although the example of using Nd:YAG as the laser medium is described above, the disclosure is not limited thereto and other media such as Alexandrite may also be used. Instead of using the laser medium, a dye laser (e.g., a dye laser using rhodamine 6G as a gain medium) that generates light having a wavelength within a range from 575 nm to 585 nm or a gas laser (e.g., a copper vapor laser) that generates light having a wavelength of 578 nm may be employed to generate the treatment light.

Referring back to FIG. 2, one side of the light generating unit 100 is directly/indirectly connected to the light irradiation unit 200 of the handpiece by an optical device 114 inside the main body and the light transmission portion 300. Therefore, the treatment light generated by the light generating unit 100 is transmitted to the light irradiation unit 200 of the handpiece along the optical path formed by the optical device and the light transmission portion 300. The light irradiation unit 200 may include a focusing optical system to focus the treatment light transmitted from the light transmission portion 300 and irradiate a treatment site with the focused treatment light. The light transmission portion 300 may include at least one optical fiber. Besides, the light transmission portion 300 may be variously implemented using a plurality of relay lenses or the like to transmit the treatment light.

Meanwhile, the setting unit 11 refers to a component that allows a user to set various options related to the operations of the light treatment apparatus. Through the setting unit 11, a user can set various parameters such as the treatment mode, the wavelength of treatment light, the pulse width of a treatment light pulse, and a spot size. The setting unit 11 may be configured as a control panel provided on the outer surface of the main body 10, or may be configured as a touchscreen display.

Meanwhile, the controller 400 refers to a component for controlling the components of the light treatment apparatus according to an embodiment. The controller 400 controls the operations of the light treatment apparatus based on the settings made by a user through the setting unit 11, the control made by the user through the control unit 21 of the handpiece, or the settings stored in its own memory (not shown).

For example, the controller 400 may control the operations of the light generating unit 100 to control the pulse pattern and pulse width of the treatment light generated by the light generating unit 100. In this case, the controller 400 controls the electric circuit connected to the flash lamp to control the excitation operation of the laser medium, thereby controlling the pulse pattern and pulse width of the treatment light. Alternatively, the pulse pattern and pulse width of the treatment light may also be controlled by controlling the optical device (e.g. the shutter) on the optical path where the treatment light travels. In this way, the controller 400 may control the light generating unit 100 and the optical device 114 on the optical path, thereby controlling various parameters such as the on/off operations of the treatment light, the output of the treatment light, the wavelength of the treatment light, and the pulse of the treatment light.

Further, the controller 400 may control the cooling operations of the cooler (not shown) in the handpiece as well as the light generating unit, or may control various treatment operations of the light generating unit or handpiece based on detection information received from various sensors of the handpiece.

Meanwhile, the light treatment apparatus 1 according to an embodiment refers to an apparatus that irradiates a skin surface with the treatment light to treat blood vessels themselves located inside skin or lesions due to the blood vessels. However, different types of treatment are required according to the sizes of blood vessels, blood flow rates, lesions, and a patient's characteristics. For example, when the blood vessels having a large diameter or a high blood flow rate are subjected to the treatment, the intensive irradiation of high-energy treatment light is required. On the other hand, when the diameters of the blood vessels are small or the walls of the blood vessels are weak, the irradiation is required to minimize damage caused by the treatment light.

According to the disclosure, a plurality of treatment modes is provided to output the treatment light having optimal parameters for various vascular lesions. In this case, the treatment light may be output in the form of a single pulse, and the pulse pattern of the treatment light output may be varied depending on the treatment modes. Here, the pulse may refer to the waveform of the treatment light, which is formed by on/off of the treatment light during the irradiation of the treatment light, and may refer to a single waveform of the treatment light output in response to a single firing action made by a user.

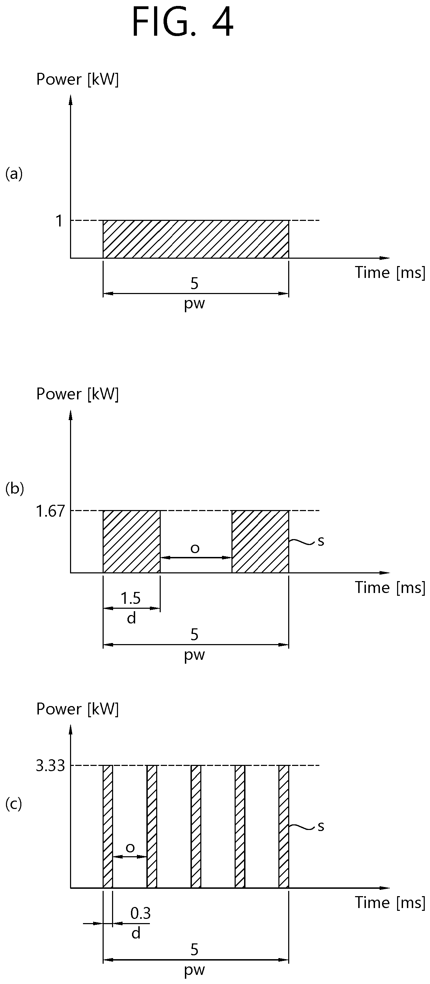

FIG. 4 is a graph showing the pulse patterns of the treatment light according to the plurality of treatment modes. In FIG. 4, (a) to (c) show single pulse patterns of treatment light in the respective treatment modes, in which the treatment light in each treatment mode has a pulse width PW of 5 ms and an energy of 5J is delivered per pulse. In the light treatment apparatus according to an embodiment, the treatment modes include a single pulse mode and a sub-pulse mode.

The single pulse mode refers to a treatment mode in which one pulse of the treatment light is continuously generated as shown in (a) of FIG. 4. In the single pulse mode, the pulse of the treatment light is constant having similar intensity without substantial off-time. For example, as shown in (a) of FIG. 4, the pulse of the treatment light in the single pulse mode continues for a duration of 5 ms with an intensity of 1kW, thereby delivering the energy of 5J.

The sub-pulse mode refers to a treatment mode in which one pulse of the treatment light includes a plurality of sub-pulses discretely generated as shown in (b) and (c) of FIG. 4. In the sub-pulse mode, the sub-pulse has a shorter duration d than the pulse of the treatment light, and the sub-pulses are sequentially generated discretely by a predetermined off period o.

In the sub-pulse mode, various treatment modes may be implemented by varying the duration d of the sub-pulse. For example, as shown in (b) of FIG. 4, the sub-pulses each having a duration d of 1.5 ms may make up the treatment light pulse, thereby implementing the treatment mode. Further, as shown in (c) of FIG. 4, the sub-pulses each having a duration d of 300 µs may make up the treatment light pulse, thereby implementing the treatment mode. In this case, to deliver an energy of 5J per treatment light pulse, one treatment light pulse in the treatment mode of using the sub-pulses each having the duration of 1.5 ms may include two pulses each having an intensity of about 1.67 kW. Further, one treatment light pulse in the treatment mode of using the sub-pulses each having the duration of 300 *µ*s may include five pulses each having an intensity of about 3.33 kW

In this case, the treatment in the single pulse mode as shown in (a) of FIG. 4 is performed in such a manner that energy having a relatively low intensity is continuously provided to a treatment site over a long period of time. On the other hand, the treatment in the sub-pulse mode as shown in (b) of FIG. 4 is performed in such a manner that energy having a relatively high intensity is repeatedly provided to a treatment site for a short period of time, and heat transferred to the treatment site is diffused in the off-time between the sub-pulses. In this way, even when one treatment light pulse has the same pulse width and delivers the same energy, the treatment may be performed in different ways according to the pulse patterns of the treatment light, thereby providing various treatment modes.

For example, according to an embodiment, the light treatment apparatus 1 for treating vascular lesions provides three treatment modes including a single pulse mode, a first sub-pulse mode, and a second sub-pulse mode. In the single pulse mode, the treatment light pulse is provided in the form of continuous light as shown in (a) of FIG. 4. Further, the treatment light pulses in the first and second sub-pulse modes include a plurality of sub-pulses s as shown in (b) and (c) of FIG. 4, respectively. Here, the sub-pulse s in the first sub-pulse mode may have a duration in a range from 1 ms to 2 ms, for example, a duration of 1.5 ms. Further, the sub-pulse s in the second sub-pulse mode may have a duration in a range from 100 µs to 500 µs, for example, a duration of 300 µs.

A user may select an appropriate treatment mode among the plurality of treatment modes through the setting unit 11 to perform the treatment. When the treatment mode is set, the size of a blood vessel to be treated, the blood flow rate, the location of a blood vessel, a patient's skin condition, etc. may be taken into account. For example, the blood vessels having a relatively large diameter may be treated in the single pulse mode, and the blood vessels having a relatively small diameter or located inside thin skin tissue may be treated in the sub-pulse mode. In particular, the second sub-pulse mode may be used to treat tinier capillaries or the like compared to that of the first sub-pulse mode.

Meanwhile, a user may set various parameters of the treatment light pulse as well as the treatment mode through the setting unit 11. For example, when the light generating unit 100 selectively generates light having a plurality of wavelengths, a user may select the wavelength of the treatment light through the setting unit 11. In addition, for convenience of description, FIG. 4 illustrates an example that the treatment light pulse delivers the same energy in each treatment mode, but the energy delivered by each treatment light pulse may be adjusted and set through the setting unit 11, or the energy peak upper limit of the treatment light pulse (e.g., the intensity upper limit of the sub-pulse) may be set through the setting unit 11.

The pulse width pw of the treatment light may also be changed and set through the setting unit 11. Here, the pulse width pw refers to a period of time from the starting point to the ending point of one treatment light pulse. Therefore, the pulse width pw is different from the duration d of the foregoing sub-pulse, and may be a period of time that includes the off-time o between the sub-pulses according to the treatment modes. For example, the treatment light pulse shown in (a) to (c) of FIG. 4 has a pulse width of 5 ms.

Meanwhile, an adjustable pulse width range for the treatment light may be set in each treatment mode, and a user may adjust the pulse width in consideration of a patient's lesion condition, skin characteristics, etc. even in the same treatment mode to perform the treatment. However, the pulse width of the treatment light, which is adjustable in each treatment mode, may be somewhat different for each treatment mode. For example, in the single pulse mode, adjustment is allowed up to a relatively short pulse width compared to that of the sub-pulse mode. When the treatment light pulse has an excessively short pulse width in the sub-pulse mode, not only it is difficult to implement the sub-pulses with uniform intensity due to the oscillation characteristics of the laser medium, but also the sub-pulse mode has no significant difference in treatment effect from the single pulse mode due to decrease in the off-time between the sub-pulses. Therefore, according to an embodiment, the treatment light pulse having a very short pulse width is implemented in the single pulse mode.

For example, the single pulse mode is configured to set the treatment light pulse having a pulse width of at least 0.1 ms. Further, the first and second sub-pulse modes are configured to set the treatment light pulse having a pulse width of at least 5 ms. Therefore, the treatment light pulse having a pulse width greater than or equal to 0.1 ms and shorter than 5 ms is implemented only in the single pulse mode.

Specifically, according to an embodiment, the single pulse mode is configured to adjust the pulse width of the treatment light pulse within a range from 0.1 ms to 60 ms. Further, the first and second sub-pulse modes are configured to adjust the pulse width of the treatment light pulse within a range from 5 ms to 60 ms. However, an adjustable pulse width range for each treatment mode may be varied depending on the wavelengths of the light generated by the light generating unit 100. For example, in the case of using light having a first wavelength, the single pulse mode may be adjustable in the pulse width within a range [0.1 ms, 60 ms] and the first and second sub-pulse modes may be adjustable in the pulse width within a range [5 ms, 60 ms]. In the case of using light having a second wavelength, the single pulse mode may be adjustable in the pulse width within a range [0.1 ms, 40 ms], and the first and second sub-pulse modes may be adjustable in the pulse width within a range [5ms, 40ms].

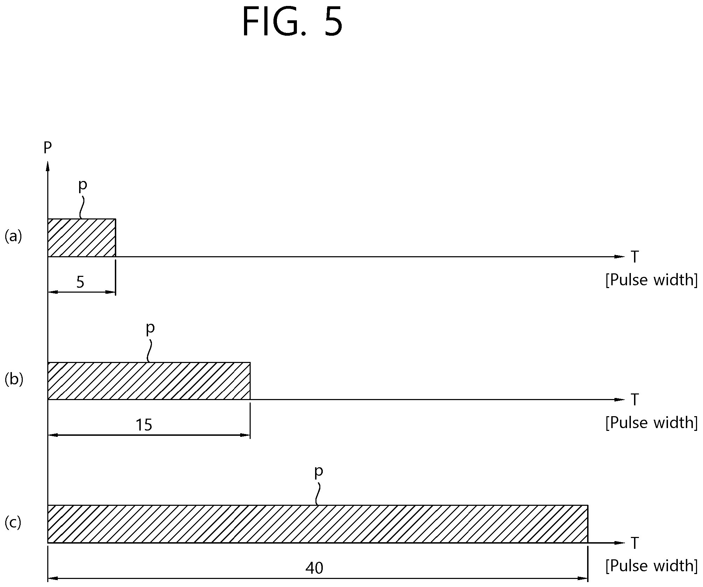

However, when the pulse width of the treatment light pulse is adjusted in the set treatment mode, the number of sub-pulses s that make up the treatment light pulse, an off-time length, the arrangement of the sub-pulses, etc. may be varied depending on the sections of the pulse width. In particular, a treatment target, a treatment lesion, and a treatment mechanism are different according to the treatment modes, and thus a pattern of the treatment light pulse varied depending on a pulse width change may be different according to the treatment modes. Below, adjustment in the pulse width of the treatment light pulse depending on the sections of the pulse width will be described with reference to FIGS. 5 to 7.

FIG. 5 is a graph showing the pulse structures of treatment light according to the pulse widths in the single pulse mode of FIG. 4. In FIG. 5, (a) to (c) are the graphs showing the treatment light pulse p set with different pulse widths in the single pulse mode. Specifically, (a) of FIG. 5 shows a pulse width of 5 ms, (b) of FIG. 5 shows a pulse width of 15 ms, and (c) of FIG. 5 shows a pulse width of 40 ms. As shown in (a) to (c) of FIG. 5, one treatment light pulse p is continuously generated in the single pulse mode, and thus the pattern of the treatment light pulse is changed similarly to change in the pulse width when the pulse width is changed.

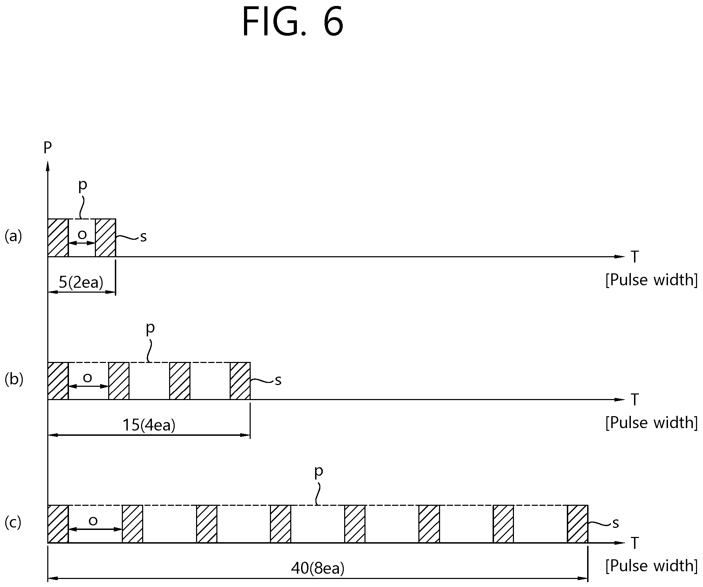

FIG. 6 is a graph showing the pulse structures of treatment light according to the pulse widths in the first sub-pulse mode of FIG. 4. Specifically, (a) to (c) of FIG. 6 show the structures of the treatment light pulse when the pulse widths are 5 ms, 15 ms, and 40 ms, respectively.

As described above, the treatment light pulse in the first sub-pulse mode includes the sub-pulses s each having a duration d of 1.5 ms. Therefore, as shown in (a) of FIG. 6, one treatment light pulse having a pulse width of 5 ms includes two sub-pulses, and an off-time of 2 ms between the sub-pulses. When the setting is made to increase the pulse width, the pulse width of the treatment light pulse may be adjusted by increasing the number of sub-pulses s that make up the treatment light pulse. In this case, the sub-pulses that make up one treatment light pulse are arranged at equidistant intervals, and thus the off-time o between the sub-pulses is changed as the pulse width is adjusted (see (b) of FIG. 6).

When treatment is performed for small blood vessels, a relatively long pulse width is needed for the treatment. However, if energy delivered by the treatment light pulse increases as the pulse width increases, skin tissue or the blood vessels to be treated may be thermally damaged. Therefore, when the set pulse width is longer than or equal to a preset value, the pulse width of the treatment light pulse p may be adjusted by increasing the off-time o between the sub-pulses without further increasing the number of sub-pulse s included in the treatment light pulse p.

For example, the first sub-pulse mode according to an embodiment is configured to adjust the pulse width within a range from 5 ms to 60 ms. Here, the range from 5 ms to 30 ms forms a first pulse width section, and the range from 30 ms to 60 ms forms a second pulse width section. In the first pulse width section, the number of sub-pulses included in the treatment light pulse increases in sequence as the set pulse width increases. Further, when the pulse width is adjusted in the second pulse width section, the pulse width of the treatment light pulse p may be changed in such a manner that the off-time o between the sub-pulses s increases while the number of sub-pulses is maintained the same as the maximum number of sub-pulses of the first pulse width section. For example, the number of sub-pulses s in the first pulse width section increases from a minimum of 2 to a maximum of 8 as the pulse width increases, but the number of sub-pulses s in the second pulse width section is maintained at 8 even though the pulse width increases (see (c) of FIG. 6). Therefore, in the first pulse width section, energy delivered per unit time is maintained within a preset range even when the pulse with increases. On the other hand, in the second pulse width section, energy delivered per unit time decreases as the pulse width increases.

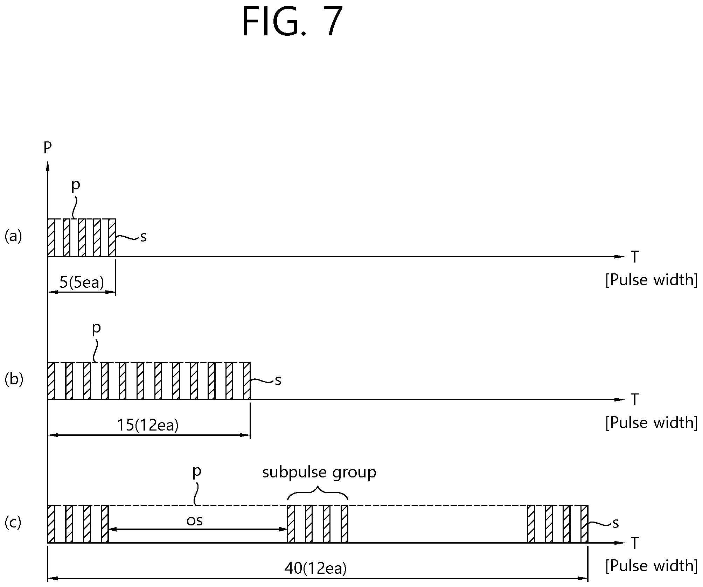

FIG. 7 is a graph showing the pulse structures of treatment light according to the pulse widths in the second sub-pulse mode of FIG. 4. Specifically, (a) to (c) of FIG. 7 show the structures of the treatment light pulse when the pulse widths are 5 ms, 15 ms, and 40 ms, respectively.

As described above, the treatment light pulse p in the second sub-pulse mode includes the sub-pulses s each having a duration of 300 µs. Therefore, as shown in (a) of FIG. 7, one treatment light pulse p having a pulse width of 5 ms includes five sub-pulses s, and an off-time of 0.875 ms between the sub-pulses s. When the setting is made to increase the pulse width, the pulse width of the treatment light pulse may be adjusted by changing the number of sub-pulses, which make up the treatment light pulse, and the arrangement of the sub-pulses.

The foregoing first sub-pulse mode includes the first pulse width section in which the number of sub-pulses increases as the pulse width is changed, and the second pulse width section in which the off-time increases without further increasing the number of sub-pulses as the pulse width is changed. The pulse width, which can be set in the second sub-pulse mode, is also adjusted in such a manner that the number of sub-pulses s increases within the preset pulse width range, but adjusted in a pulse width section above the preset value (a third pulse width section) while the number of sub-pulses is maintained the same.

However, the sub-pulses are arranged at equidistant intervals in the second pulse section of the first sub-pulse mode, but the pulse width in the third pulse width section of the second sub-pulse mode is adjusted in such a manner that a plurality of sub-pulses forms a predetermined sub-pulse group and an off-time os between the sub-pulse groups is changed. When the duration of the sub-pulse is given in units of *µ*s like that in the second sub-pulse mode, it is easier to control the irradiation timing of each sub-pulse mode as a sub-pulse group rather than individually, and the individual control and the group control showed similar results in terms of treatment effectiveness.

Therefore, the second sub-pulse mode according to an embodiment is configured to adjust the pulse width within a range from 5 ms to 60 ms, in which the range from 5 ms to 15 ms forms a first pulse width section, and the range from 15 ms to 60 ms forms a third pulse width section. In the first pulse width section, the number of sub-pulses s included in the treatment light pulse p increases in sequence as the set pulse width increases. Further, when the pulse width is adjusted in the third pulse width section, the sub-pulses are generated as grouped according to preset patterns, and the pulse width of the treatment light pulse p may be changed in such a manner that an off-time os between the sub-pulse groups is changed.

For example, the number of sub-pulses s in the first pulse width section increases from a minimum of 5 to a maximum of 12 as the pulse width increases (see (a) and (b) of FIG. 7), but the number of sub-pulses s in the third pulse width section is maintained at 12 even though the pulse width increases (see (c) of FIG. 7). In this case, as shown in (c) of FIG. 7, in the third pulse width section, four sub-pulses s make up one sub-pulse group, and one treatment light pulse includes three sub-pulse groups irradiated with a constant off-time os therebetween. Further, when the pulse width is adjusted in the third pulse width section, the pulse width may be changed in such a manner that the off-time os between the sub-pulse groups is changed while the number of sub-pulses, the number of sub-pulse groups, and the duration of one sub-pulse group are maintained the same. Therefore, even in the second pulse width section, energy delivered per unit time is maintained within a preset range even when the pulse with increases. On the other hand, in the third pulse width section, energy delivered per unit time decreases as the pulse width increases.

Although the plurality of treatment modes to provide various treatment modes for vascular lesions and various pattern of the treatment light pulse according to the pulse widths in the treatment modes have been described above, the foregoing descriptions are merely examples, and the disclosure is not limited thereto

For example, the first sub-pulse mode according to an embodiment is configured to have the first pulse width section and the second pulse width section, and the second sub-pulse mode is configured to have the first pulse width section and the third pulse width section. However, both the first and second sub-pulse modes may be equally implemented to have the first pulse width section and the second pulse width section, or have the first pulse width section and the third pulse width section.

Further, according to an alternative embodiment, a light treatment apparatus that selectively generates light having a plurality of wavelengths to perform treatment may be configured to have the same pulse width sections as those of the foregoing embodiments in a treatment mode where light having a first wavelength (e.g., 1064 nm) is irradiated, but may be configured to have the first pulse width section and the third pulse width section in the first sub-pulse mode and to have the first pulse width section and the second pulse width section in the second sub-pulse mode unlike those of the foregoing embodiments in a treatment mode where light having a second wavelength (e.g., 532 nm) is irradiated.

In this way, the light treatment apparatus for treating vascular lesions according to the disclosure is configured to set energy delivery methods variously according to the pattern and the pulse width of the treatment light pulse, thereby performing optimal treatment under various treatment conditions.

According to the disclosure, the treatment light having various pulse waveforms is irradiated by one apparatus, and thus effective treatment is possible using the treatment light having an appropriate pulse waveform in consideration of the sizes of blood vessels, the locations of lesions, a patient's skin conditions, etc.

Although a few embodiments of the disclosure have been described above in detail, the disclosure is not limited to the foregoing embodiments. It will be apparent to a person having ordinary knowledge in the art to which the disclosure pertains that various changes or modifications can be made without departing from the scope of the technical features of the disclosure defined by the appended claims.

## Claims

1. A light treatment apparatus configured for the treatment of vascular lesions on skin via skin from the outside, the apparatus comprising:
a light generating unit (100), wherein the light generating unit is a laser configured to generate light in one of the wavelength ranges 530-535 nm, 575-585 nm, and 1060-1070 nm;
a setting unit (11) configured to set a pulse pattern of treatment light generated in the light generating unit, and wherein the setting unit is configured to adjust the pulse width of the treatment light; and
a handpiece (20) configured to receive the treatment light generated in the light generating unit and irradiate a treatment site with light;
wherein a single pulse mode for irradiating treatment light comprising a single pulse (p), and a sub-pulse mode for irradiating treatment light comprising a plurality of sub-pulses (s) are set through the setting unit,
wherein the pulse width of the treatment light is adjusted as a pulse width of 0.1 ms or more in the single pulse mode, and the pulse width of the treatment light is adjusted as a pulse width of 5 ms or more in the sub-pulse mode,
wherein a pulse width section selectable in the sub-pulse mode comprises a first pulse width section where the pulse width is adjustable by changing the number of sub-pulses forming the treatment light pulse, and a second pulse width section where the pulse width is adjustable by changing an off-time between the sub-pulses while maintaining the number of sub-pulses forming the treatment light pulse,
wherein the handpiece comprises a cooler to protect a patient's skin from thermal damage during treatment, and wherein
at the end of the handpiece there is provided a light irradiation unit (200) comprising a focusing optical system configured to focus the treatment light and irradiate a treatment site with the focused treatment light.

2. The apparatus of claim 1, wherein an adjustable range for the pulse width of the treatment light in the single pulse mode is configured to allow adjustment up to a shorter pulse width compared to an adjustable range for the pulse width of the treatment light in the sub-pulse mode.

3. The apparatus of claim 1, wherein the number of sub-pulses in the second pulse width section is equal to a maximum number of sub-pulses in the first pulse width section.

4. The apparatus of claim 1, wherein
the pulse width section selectable in the sub-pulse mode further comprises a third pulse width section where the pulse width is adjusted as an off-time between sub-pulse groups is changed while sub-pulses that make up the treatment light pulse are irradiated as a plurality of sub-pulse groups.

5. The apparatus of claim 4, wherein the number of sub-pulses in the third pulse width section is equal to a maximum number of sub-pulses in the first pulse width section.

6. The apparatus of claim 1, wherein the sub-pulse mode comprises a first sub-pulse mode with a plurality of sub-pulses having a pulse width within a range from 1 to 3 ms, and a second sub-pulse mode with a plurality of sub-pulses having a pulse width within a range from 100 to 700 *µ*s.

7. The apparatus of claim 6, wherein energy delivered to a treatment site per unit time in the first pulse width section is maintained within a preset range, and energy delivered to a treatment site per unit time in the second pulse width section decreases as the pulse width increases.

8. The apparatus of claim 6, wherein a pulse width section selectable in the second sub-pulse mode comprises the first pulse width section and a third pulse width section where the pulse width is adjusted as an off-time between sub-pulse groups is changed while sub-pulses that make up the treatment light pulse are irradiated as a plurality of sub-pulse groups.

9. The apparatus of claim 8, wherein energy delivered to a treatment site per unit time in the first pulse width section is maintained within a preset range, and energy delivered to a treatment site per unit time in the third pulse width section decreases as the pulse width increases.

## Patentansprüche

1. Lichtbehandlungsvorrichtung, die zur Behandlung von Gefäßläsionen auf der Haut von außen durch die Haut hindurch ausgelegt ist, wobei die Vorrichtung aufweist:
eine Lichterzeugungseinheit (100), wobei die Lichterzeugungseinheit ein Laser ist, der dazu ausgelegt ist, in einem der Wellenlängenbereiche 530-535 nm, 575-585 nm und 1060-1070 nm Licht zu erzeugen,
eine Einstelleinheit (11), die dazu ausgelegt ist, ein Pulsmuster des in der Lichterzeugungseinheit erzeugten Behandlungslichts einzustellen, und wobei die Einstelleinheit dazu ausgelegt ist, die Pulsbreite des Behandlungslichts einzustellen, und
ein Handstück (20), das dazu ausgelegt ist, das in der Lichterzeugungseinheit erzeugte Behandlungslicht aufzunehmen und eine Behandlungsstelle mit Licht zu bestrahlen,
wobei über die Einstelleinheit ein Einzelpulsmodus zum Bestrahlen mit Behandlungslicht, das einen einzelnen Puls (p) aufweist, und ein Subpulsmodus zum Bestrahlen mit Behandlungslicht, das eine Vielzahl von Subpulsen (s) aufweist, eingestellt werden,
wobei die Pulsbreite des Behandlungslichts im Einzelpulsmodus auf eine Pulsbreite von 0,1 ms oder mehr eingestellt wird und die Pulsbreite des Behandlungslichts im Subpulsmodus auf eine Pulsbreite von 5 ms oder mehr eingestellt wird,
wobei ein im Subpulsmodus wählbarer Pulsbreitenabschnitt einen ersten Pulsbreitenabschnitt aufweist, in dem die Pulsbreite durch Ändern der Anzahl der den Behandlungslichtpuls bildenden Subpulse einstellbar ist, sowie einen zweiten Pulsbreitenabschnitt, in dem die Pulsbreite durch Ändern einer Ausschaltzeit zwischen den Subpulsen einstellbar ist, während die Anzahl der den Behandlungslichtpuls bildenden Subpulse beibehalten wird,
wobei das Handstück einen Kühler aufweist, um die Haut eines Patienten während der Behandlung vor thermischen Schäden zu schützen, und
wobei am Ende des Handstücks eine Lichtbestrahlungseinheit (200) vorgesehen ist, die ein optisches Fokussiersystem aufweist, das dazu ausgelegt ist, das Behandlungslicht zu fokussieren und eine Behandlungsstelle mit dem fokussierten Behandlungslicht zu bestrahlen.

2. Vorrichtung nach Anspruch 1, wobei ein Einstellbereich für die Pulsbreite des Behandlungslichts im Einzelpulsmodus so ausgelegt ist, dass eine Einstellung bis zu einer kürzeren Pulsbreite im Vergleich zu einem Einstellbereich für die Pulsbreite des Behandlungslichts im Subpulsmodus möglich ist.

3. Vorrichtung nach Anspruch 1, wobei die Anzahl der Subpulse im zweiten Pulsbreitenabschnitt gleich einer maximalen Anzahl von Subpulsen im ersten Pulsbreitenabschnitt ist.

4. Vorrichtung nach Anspruch 1, wobei der im Subpulsmodus wählbare Pulsbreitenabschnitt ferner einen dritten Pulsbreitenabschnitt aufweist, in dem die Pulsbreite angepasst wird, indem eine Ausschaltzeit zwischen Subpulsgruppen verändert wird, während die den Behandlungslichtpuls bildenden Subpulse als eine Vielzahl von Subpulsgruppen abgestrahlt werden.

5. Vorrichtung nach Anspruch 4, wobei die Anzahl der Subpulse im dritten Pulsbreitenabschnitt gleich der maximalen Anzahl von Subpulsen im ersten Pulsbreitenabschnitt ist.

6. Vorrichtung nach Anspruch 1, wobei der Subpulsmodus einen ersten Subpulsmodus mit einer Vielzahl von Subpulsen aufweist, deren Pulsbreite in einem Bereich von 1 bis 3 ms liegt, und einen zweiten Subpulsmodus mit einer Vielzahl von Subpulsen, deren Pulsbreite in einem Bereich von 100 bis 700 µs liegt.

7. Vorrichtung nach Anspruch 6, wobei die pro Zeiteinheit an eine Behandlungsstelle abgegebene Energie im ersten Pulsbreitenabschnitt innerhalb eines voreingestellten Bereichs gehalten wird und die pro Zeiteinheit an eine Behandlungsstelle abgegebene Energie im zweiten Pulsbreitenabschnitt mit zunehmender Pulsbreite abnimmt.

8. Vorrichtung nach Anspruch 6, wobei ein im zweiten Subpulsmodus wählbarer Pulsbreitenabschnitt den ersten Pulsbreitenabschnitt und einen dritten Pulsbreitenabschnitt aufweist, in welchem die Pulsbreite angepasst wird, indem eine Ausschaltzeit zwischen Subpulsgruppen verändert wird, während die den Behandlungslichtpuls bildenden Subpulse als eine Vielzahl von Subpulsgruppen abgestrahlt werden.

9. Vorrichtung nach Anspruch 8, wobei die pro Zeiteinheit an eine Behandlungsstelle abgegebene Energie im ersten Pulsbreitenabschnitt innerhalb eines voreingestellten Bereichs gehalten wird und die pro Zeiteinheit an eine Behandlungsstelle abgegebene Energie im dritten Pulsbreitenabschnitt mit zunehmender Pulsbreite abnimmt.

## Revendications

1. Appareil de traitement par lumière configuré pour le traitement de lésions vasculaires cutanées, à travers la peau, depuis l'extérieur, l'appareil comprenant :
une unité de production de lumière (100), dans laquelle l'unité de production de lumière est un laser configuré pour produire une lumière dans l'une des plages de longueurs d'onde 530-535 nm, 575-585 nm et 1060-1070 nm ;
une unité de réglage (11) configurée pour régler un motif d'impulsions de la lumière de traitement produite dans l'unité de production de lumière, et dans laquelle l'unité de réglage est configurée pour ajuster la largeur d'impulsion de la lumière de traitement ; et
une pièce à main (20) configurée pour recevoir la lumière de traitement produite dans l'unité de production de lumière et irradier un site de traitement avec la lumière ;
dans lequel un mode d'impulsion unique pour irradier une lumière de traitement comprenant une impulsion unique (p), et un mode de sous-impulsions pour irradier une lumière de traitement comprenant une pluralité de sous-impulsions (s) sont réglés par l'intermédiaire de l'unité de réglage,
dans lequel la largeur d'impulsion de la lumière de traitement est ajustée à une largeur d'impulsion de 0,1 ms ou plus dans le mode d'impulsion unique, et la largeur d'impulsion de la lumière de traitement est ajustée à une largeur d'impulsion de 5 ms ou plus dans le mode de sous-impulsions,
dans lequel une section de largeur d'impulsion pouvant être sélectionnée dans le mode de sous-impulsions comprend une première section de largeur d'impulsion où la largeur d'impulsion est ajustable en modifiant le nombre de sous-impulsions formant l'impulsion de lumière de traitement, et une deuxième section de largeur d'impulsion où la largeur d'impulsion est ajustable en modifiant un temps d'arrêt entre les sous-impulsions tout en maintenant le nombre de sous-impulsions formant l'impulsion de lumière de traitement,
dans lequel la pièce à main comprend un refroidisseur pour protéger la peau d'un patient contre un dommage thermique pendant le traitement, et dans lequel, à l'extrémité de la pièce à main, est prévue une unité d'irradiation de lumière (200) comprenant un système optique de focalisation configuré pour focaliser la lumière de traitement et irradier un site de traitement avec la lumière de traitement focalisée.

2. Appareil selon la revendication 1, dans lequel une plage ajustable pour la largeur d'impulsion de la lumière de traitement dans le mode d'impulsion unique est configurée pour permettre un ajustement jusqu'à une largeur d'impulsion plus courte par rapport à une plage ajustable pour la largeur d'impulsion de la lumière de traitement dans le mode de sous-impulsions.

3. Appareil selon la revendication 1, dans lequel le nombre de sous-impulsions dans la deuxième section de largeur d'impulsion est égal à un nombre maximal de sous-impulsions dans la première section de largeur d'impulsion.

4. Appareil selon la revendication 1, dans lequel la section de largeur d'impulsion pouvant être sélectionnée dans le mode de sous-impulsions comprend en outre une troisième section de largeur d'impulsion où la largeur d'impulsion est ajustée en modifiant un temps d'arrêt entre des groupes de sous-impulsions tandis que des sous-impulsions constituant l'impulsion de lumière de traitement sont irradiées sous la forme d'une pluralité de groupes de sous-impulsions.

5. Appareil selon la revendication 4, dans lequel le nombre de sous-impulsions dans la troisième section de largeur d'impulsion est égal à un nombre maximal de sous-impulsions dans la première section de largeur d'impulsion.

6. Appareil selon la revendication 1, dans lequel le mode de sous-impulsions comprend un premier mode de sous-impulsions comportant une pluralité de sous-impulsions ayant une largeur d'impulsion comprise à l'intérieur d'une plage de 1 à 3 ms, et un second mode de sous-impulsions comportant une pluralité de sous-impulsions ayant une largeur d'impulsion comprise à l'intérieur d'une plage de 100 à 700 µs.

7. Appareil selon la revendication 6, dans lequel l'énergie délivrée à un site de traitement par unité de temps dans la première section de largeur d'impulsion est maintenue à l'intérieur d'une plage prédéfinie, et l'énergie délivrée à un site de traitement par unité de temps dans la deuxième section de largeur d'impulsion diminue à mesure que la largeur d'impulsion augmente.

8. Appareil selon la revendication 6, dans lequel une section de largeur d'impulsion pouvant être sélectionnée dans le second mode de sous-impulsions comprend la première section de largeur d'impulsion et une troisième section de largeur d'impulsion dans laquelle la largeur d'impulsion est ajustée en modifiant un temps d'arrêt entre des groupes de sous-impulsions tandis que des sous-impulsions constituant l'impulsion de lumière de traitement sont irradiées sous la forme d'une pluralité de groupes de sous-impulsions.

9. Appareil selon la revendication 8, dans lequel l'énergie délivrée à un site de traitement par unité de temps dans la première section de largeur d'impulsion est maintenue à l'intérieur d'une plage prédéfinie, et l'énergie délivrée à un site de traitement par unité de temps dans la troisième section de largeur d'impulsion diminue à mesure que la largeur d'impulsion augmente.
